# EUROPEAN PATENT SPECIFICATION

(11) **EP 2 756 099 B1**
(45) Date of publication and mention of the grant of the patent: **28.06.2017**
(21) Application number: 12783347.3
(22) Date of filing: 13.09.2012
(51) Int. Cl.: C12Q 1/68

(54) **Method to detect HPV-induced cervical cancer**
Verfahren zum Nachweis von HPV-induzierten Gebärmutterhalskrebs
Méthode de détection du cancer du col de l'utérus

(30) Priority: 15.09.2011 EP 11181492
(43) Date of publication of application: 23.07.2014
(73) Proprietor: Self-screen B.V., 1098 RX Amsterdam (NL)
(72) Inventor: SNIJDERS, Petrus Josephus Ferdinandus, NL-1087 EL Amstelveen (NL); STEENBERGEN, Renske Daniëla Maria, NL-1432 PH Aalsmeer (NL); HEIDEMAN, Daniëlle Anne Marie, NL-1079 XA Amsterdam (NL); MEIJER, Christophorus Joannes Lambertus Maria, NL-1081 HV Amsterdam (NL)
(74) Representative: V.O.
(86) International application number: PCT/NL2012/050645
(87) International publication number: WO 2013/039394

(56) References cited:
- WO-A1-2009/036332
- WO-A1-2009/128714
- WILTING SASKIA M ET AL: "Methylation-mediated silencing and tumour suppressive function of hsa-miR-124 in cervical cancer", MOLECULAR CANCER, BIOMED CENTRAL, LONDON, GB, vol. 9, no. 1, 26 June 2010 (2010-06-26), page 167, XP021077905, ISSN: 1476-4598, DOI: 10.1186/1476-4598-9-167
- BOTEZATU A ET AL: "Quantitative analysis of the relationship between microRNA-124a, -34b and -203 gene methylation and cervical oncogenesis", MOLECULAR MEDICINE REPORTS 2011 SPANDIDOS PUBLICATIONS LTD. GRC LNKD- DOI:10.3892/MMR.2010.394, vol. 4, no. 1, January 2011 (2011-01), pages 121-128, XP002670887, ISSN: 1791-2997
- RENÉE M. OVERMEER ET AL: "Combined CADM1 and MAL promoter methylation analysis to detect (pre-)malignant cervical lesions in high-risk HPV-positive women", INTERNATIONAL JOURNAL OF CANCER, vol. 129, no. 9, 28 December 2010 (2010-12-28), pages 2218-2225, XP055021025, ISSN: 0020-7136, DOI: 10.1002/ijc.25890

## Description

### FIELD OF THE INVENTION

The invention relates to the field of cancer prevention and medical diagnostics; and is concerned with a molecular diagnostic assay for human papillomavirus (HPV)-induced invasive cancers and high-grade precursor lesions thereof, such as invasive cervical cancer and premalignant cervical lesions. In particular, the present invention relates to the use of a combined analysis of MAL and hsa-miR124 promoter methylation on (hrHPV-positive) self-sampled specimens in an assay for hrHPV-induced premalignant lesions with invasive potential and hrHPV-induced invasive cancers.

### BACKGROUND OF THE INVENTION

Cancer of the uterine cervix is the second most common cancer in women world-wide and is responsible for approximately 250.000 cancer deaths a year.

Cervical squamous cell carcinoma development is characterized by a sequence of premalignant lesions, so-called cervical intraepithelial neoplasia (CIN), which are graded 1 to 3, referring to mild dysplasia (CIN 1), moderate dysplasia (CIN 2) and severe dysplasia/carcinoma *in situ* (CIN 3), respectively. CIN 1 is also referred to as low grade squamous intraepithelial lesion (LSIL) and CIN 2 and CIN 3 together as high grade squamous intraepithelial lesion (HSIL) For cervical adenocarcinoma, adenocarcinoma in situ (ACIS) is an established precursor lesion. In principle, these premalignant lesions are reversible, although the more severe the lesion, the lower the chance of spontaneous regression. Cervical cancer is considered a preventable disease because the premalignant stages can be detected by exfoliative cytology and treated relatively easily when necessary, with only minor side effects. Cervical screening is aimed to early diagnose the high-grade premalignant (i.e., CIN 2/3 and adenocarcinoma in situ) and treatable cancerous lesions, thereby reducing the mortality of invasive cervical cancer. General medical practice comprises the treatment of all women with morphologically confirmed CIN 2 , CIN 3 and adenocarcinoma in situ, in order to prevent the development of cervical cancer.

Over the past decade it has been well established that cervical carcinogenesis is initiated by an infection with high-risk human papillomavirus (hrHPV). Expression of the viral oncogenes E6 and E7, which disturb the p53 and Rb tumor suppressor pathways, respectively, has been shown to be essential for both the onset of oncogenesis and the maintenance of a malignant phenotype. Therefore, testing for hrHPV appeared as an attractive, primary screening tool. However, consistent with a multistep process of carcinogenesis, additional alterations in the host cell genome are required for progression of an hr-HPV infected cell to an invasive cancer cell. Only a small proportion of women infected with high-risk HPV will develop high-grade premalignant cervical lesions (CIN 2/3) and, if left untreated, cervical cancer. In most women with premalignant cervical lesions the lesions regress spontaneously. Of the women who participate in population based screening in The Netherlands about 5-6% have a positive hrHPV test (Bulkmans et al., Int J Cancer 2004,110:94-101). However, only at maximum 20% of them (1% of the participating women) have ≥CIN 2/3. Therefore, primary screening by hrHPV testing will be accompanied with a substantial number of redundant follow-up procedures and unnecessary anxiety amongst women, unless markers can be applied to the cervical smears that allow stratification of hrHPV positive women for risk of ≥CIN 2/3 and ≥adenocarcinoma in situ.
Large population-based screening programmes have been set-up in various countries based on physician-taken smears. However, not all women called upon will show up for having a cervical smear taken. A number of non-shows is caused by reluctance of women to have a cervical smear taken by a nurse or a physician. Studies have shown that this can be overcome by offering these women a device for self-collection of (cervico-)vaginal material at home, that can be sent to the laboratory for HPV analysis. As such, self-sampling increases participation of non-responders in current screening programs (Gök et al.2010 Brit.Medical J.;340:c1040). Self-sampling is an inexpensive and well-accepted method for cervical screening. Even more, because HPV analysis can be as good on self-collected samples as physician-collected samples for the detection of CIN2+/3+, self-sampling might become an alternative method for primary cervical cancer screening in the future. Thus, in the forthcoming years, self-sampling of (cervico-)vaginal material will become an important tool in the screening for cervical cancer.

A major challenge is to reduce the percentage of hrHPV positive women to those that have clinically meaningful lesions. Whereas cytology can serve as a secondary (so-called triage) test for hrHPV positive women on conventional scrapings, cytology is not an option for self-sampled cervico-vaginal specimens that can be taken at home, since these are not representative for the cytological status of the cervix (Brink et al., 2006, J. Clin. Microbiol. 44:2518-2523). Therefore, there is a need for supplementary or alternative triage tools to stratify hrHPV positive women into those with and without ≥CIN 2/3 and ≥adenocarcinoma in situ.

It has been shown earlier (WO 2009/128714) that combined testing for promoter methylation of the gene encoding T-lymphocyte maturation associated protein, also known as T-cell differentiation protein (further referred to as *MAL*; Genbank Accession NM_002371.2) and the gene encoding cell adhesion molecule 1 (CADM1) appeared an attractive alternative molecular triage tool when applied to physician-collected cervical scrapes that performed equally well as cytology (Hesselink et al. 2011 Clin Cancer Res. 17(8):2459-65). However, this panel performed less well on self-sampled cervico-vaginal specimens, mainly because signals for CADM1 methylation in self-samples of women with ≥CIN 2/3 were more frequently below the assay threshold than in case of physician-collected cervical samples. Although a combination of MAL or CADM1 with detection of hsa-miR-124 methylation-based silencing was suggested by Wilting et al., (Mol. Cancer 9(1):167, 2010) no prediction of possible improvements in hrHPV testing has been made.

Eijsink, J.J.H. et al. (Gynecologic Oncol. 120(2):280-283, 2011) disclosed that methylation analysis in self-sampled cervico-vaginal lavages provide a diagnostic performance comparable to detection through cytomorphology and hrHPV. However, this document does not concern a population-based screening but only a referral risk population of women in an outpatient clinic setting, in the absence of negative controls. This casts doubt on the feasibility of a population-based screening based on self-obtained samples.

Therefore, there is still need for an assay that can be performed on self-samples and that can give a more reliable indication of the presence of or the risk for developing cervical lesions.

### SUMMARY OF THE INVENTION

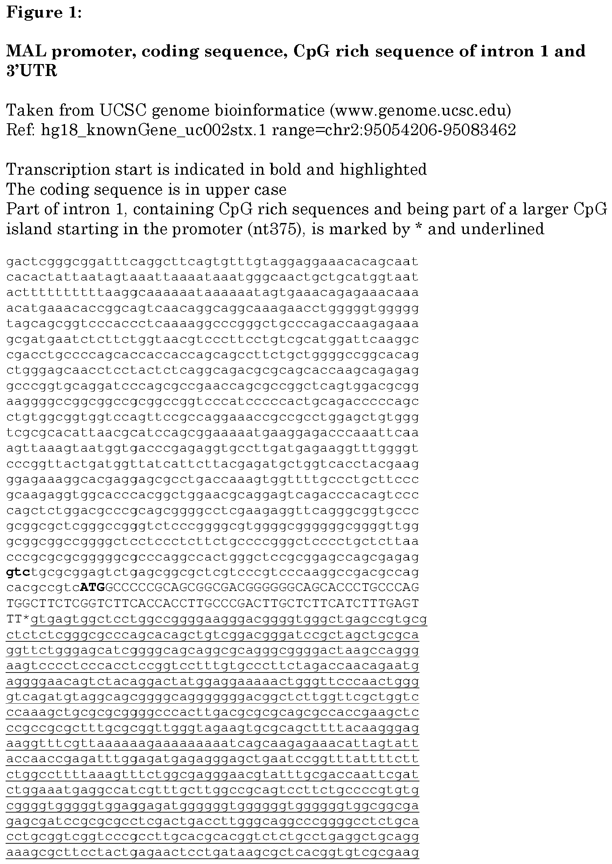

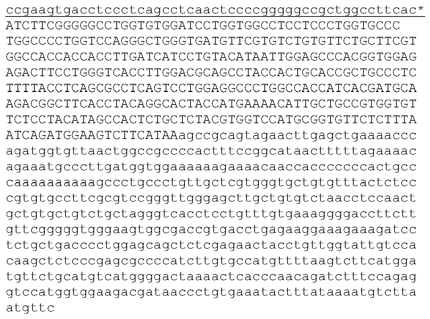

It has now surprisingly been found that combined testing for promoter methylation of the gene encoding T-lymphocyte maturation associated protein, also known as T-cell differentiation protein (further referred to as *MAL*; Genbank Accession NM_002371.2) and the microRNA hsa-miR124 (Accession number MIMAT0000422), encoded by 3 individual premature microRNA sequences located at three different genomic regions, i.e. HSA-MIR124-1 (Accession number MI0000443) at chromosome 8p23.1, HSA-MIR124-2 (Accession number MI0000444) at chromosome 8q12.3 and HSA-MIR124-3 (Accession number MI0000445) at chromosome 20q12.33 (Accession numbers as indicated on the miRNA database, miRBase (www.mirbase.org), Faculty of Life Sciences, University of Manchester), both of which have tumor suppressor properties in cervical cancer cells, provides a valuable assay to diagnose invasive cervical cancer and the high-grade precursor lesions thereof when applied to self-sampled lavage specimens testing positive for hrHPV. Accordingly, the invention relates to a method of detecting the occurrence of HPV-induced high-grade precancerous lesions and/or HPV-induced invasive cancers comprising assaying a cervico-vaginal lavage sample for the presence of an alteration in methylation of T-lymphocyte maturation associated protein (MAL) and hsa-miR124, wherein said alteration indicates the presence of HPV-induced precursor lesions with invasive potential and/or HPV-induced invasive cancers. Preferably, in said method, said HPV-induced invasive cancer is a high-risk HPV-induced invasive cancer.
In another embodiment in said method, said alteration is an increase in methylation in comparison to a normal, control sample. Preferably in the method of the invention, the methylation is assayed on the nucleic acid encoding the MAL polypeptide and regulatory regions thereof and the hsa-miR124 sequence, wherein said nucleic acid preferably is DNA. In a further preferred embodiment the assay is performed with a restriction endonuclease, preferably a methylation sensitive restriction endonuclease and also preferred is that the reagent is a nucleic acid probe or primer that binds to the nucleic acid, preferably wherein said nucleic acid probe or primer has a detectable label. Especially preferred is a nucleic acid probe which has a nucleotide sequence selected from the group consisting of the sequences depicted in Table 1.
Also part of the invention is the use of a molecular diagnostic marker set for the detection of HPV-induced high-grade precancerous lesion or HPV-induced invasive carcinoma in a cervico-vaginal lavage sample, wherein said marker set indicates an alteration in *MAL* gene or promoter methylation and an alteration in methylation of the hsa-miR124 sequence.
Further comprised in the invention is the use of a kit of parts in a method of detecting HPV-induced high- grade precancerous lesion or HPV-induced invasive carcinoma in test cells from a cervico-vaginal lavage of a subject, said kit comprising a methylation sensitive restriction endonuclease and
- means for the detection of methylation of the MAL gene or promoter wherein said means comprise probes, primers specific for MAL or specific for the MAL nucleotide sequence of Figure 1; and
- means for the detection of hsa-miR124 methylation, wherein said means comprise probes, primers specific for hsa-miR124.,
- optionally, means for the detection of HPV infection, wherein said means comprise probes and primers specific for HPV, and
- optionally means for taking a cervico-vaginal lavage. As specified herein, said means are preferably selected from the group of a VibaBrush (Rovers Medical Devices, Oss, the Netherlands) and a Pantarhei sampler (Pantarhei Devices, Zeist, The Netherlands).

### BRIEF DESCRIPTION OF THE DRAWINGS

**Figure 1** shows the *MAL* 5' regulatory region, coding sequence, CpG rich part of first intronic sequence and transcribed 3' non-coding sequences.
**Figure 2** shows the 5' regulatory regions and coding sequences of the three miR124 genomic regions, A: hsa-miR124-1, B: hsa-miR124-2, C: hsa-miR124-3
   Sequences in italic/bold are coding sequences of premature microRNAs. Underlined sequence comprises the mature microRNA sequence. The CpG rich area is marked in grey. The wild type sequence is shown (upper row) as well as the modified sequence following bisulfite treatment (lower row).
   ++ represents CpG sites, :::: indicates non-CpG 'C' converted to 'T'.

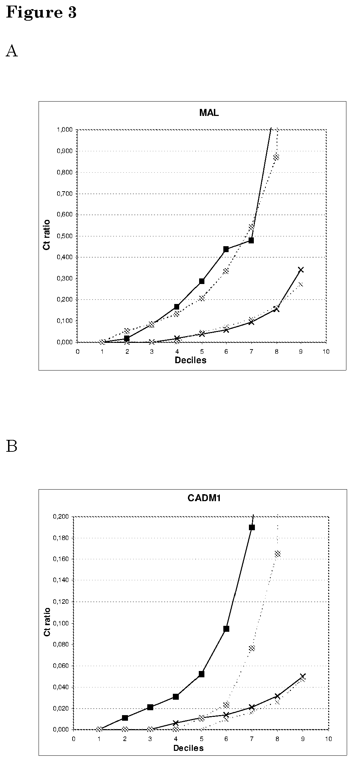

**Figure 3** shows the comparison of the Ct ratio values of the deciles of MAL (A) and CADM1 (B) methylation analysis in self-samples of women with CIN3+ (dotted line with filled squares) and women with at maximum CIN2 (dotted line with cross) and physician-taken cervical scrapes of women with CIN3+ (solid line with filled squares) and women with at maximum CIN2 (solid line with cross). This figure indicates that for MAL the signals for women with CIN3+ in self-sampled specimens are equal to those in physician-taken smears, whereas those of CADM1 for women with CIN3+ are lower as compared to those in physician-taken smears, and fall for a large part in the same range as the levels obtained in women without cervical disease.

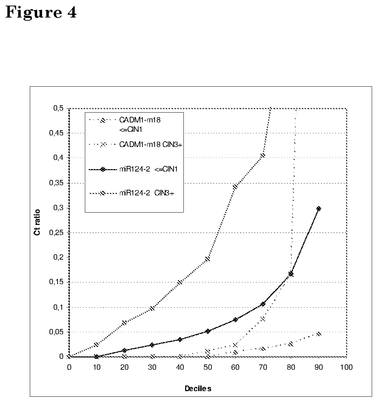

**Figure 4** shows the comparison of the Ct ratio values of the deciles of CADM1 (dotted lines) and hsa-miR124-2 (solid lines) methylation analysis in self-samples of women with CIN3+ (CADM1: cross; hsa-miR124-2: diamond) and women with ≤CIN1 (CADM1: triangle; hsa-miR124-2: square), respectively. This figure indicates that for hsa-miR124-2 the signals for women with CIN3+ in self-sampled specimens are much higher than those for CADM1. Moreover, hsa-miR124-2 methylation shows a better discrimination between self-sampled specimens of women with CIN3+ and women with ≤CIN1 compared to CADM1.

### DETAILED DESCRIPTION OF THE INVENTION

Cervical cancer is almost exclusively associated with human papillomavirus (HPV) infection. Human papillomaviruses constitute a group of more than 100 types of viruses, as identified by variations in DNA sequence. The various HPVs cause a variety of cutaneous and mucosal diseases. HPVs are broadly classified into low-risk and high-risk types, based on their ability to induce malignant changes in infected cells. Low risk HPV types such as 1, 2, 4, 6, 11, 13 and 32 are primarily associated with benign lesions or common warts, while the high risk types, such as 16, 18, 31, 33, 35, 39, 45, 51, 52, 56, 58, 59, 66, and 68 are primarily associated with premalignant and malignant epithelial lesions. These high-risk types of HPV cause growths that are usually flat and nearly invisible, as compared with the warts caused by low-risk types, e.g. HPV-6 and HPV-11.

The term "HPV-induced invasive cancer" refers to a carcinoma induced by high-risk HPV, which invades surrounding tissue. This includes all HPV-induced carcinoma histotypes, i.e., squamous cell carcinomas, adenocarcinomas, adenosquamous carcinomas and neuroendocrine carcinomas in the cervix.

The term "invasive cervical cancer" refers to a cervical carcinoma invading surrounding tissue. This includes all carcinoma histotypes, i.e., squamous cell carcinomas, adenocarcinomas, adenosquamous cell carcinomas and neuroendocrine carcinomas in the cervix.

The terms "premalignant lesion" and "precursor lesion" refer to a stage in the multistep cellular evolution to cancer with a strongly increased chance to progress to a carcinoma. With classical morphology the pathologist is unable to predict in the individual patient which of these lesions will progress or regress. The current patent application refers to a method, which can predict invasive cancer or a high-grade precursor lesion thereof.

The term "high-grade premalignant cervical lesion" refers to a stage in the multistep cellular evolution to cervical cancer with a strongly increased chance to progress to a cervical carcinoma. The high-grade premalignant cervical lesions equal the denomination CIN 2/3 and higher (i.e. ≥CIN2).

The term "capable of specifically hybridizing to" refers to a nucleic acid sequence capable of specific base-pairing with a complementary nucleic acid sequence and binding thereto to form a nucleic acid duplex.

A "complement" or "complementary sequence" is a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'.

The term "stringent hybridization conditions" refers to hybridization conditions that affect the stability of hybrids, e.g., temperature, salt concentration, pH, formamide concentration and the like. These conditions are empirically optimised to maximize specific binding and minimize non-specific binding of the primer or the probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tₘ) for the specific sequence at a defined ionic strength and pH. The Tₘ is the temperature (under defined ionic strength and pH) at which 50% of a complementary target sequence hybridises to a perfectly matched probe or primer. Typically, stringent conditions will be those in which the salt concentration is less than about 1.0 M Na ion, typically about 0.01 to 1.0 M Na ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double-and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in polymerase chain reaction (PCR) amplification.

The term "probe" refers to a single-stranded oligonucleotide sequence that will recognize and form a hydrogen-bonded duplex with a complementary sequence in a target nucleic acid sequence analyte or its cDNA derivative.

"MicroRNAs" (miRNAs or MIRs) are short ribonucleic acid (RNA) molecules, on average only 22 nucleotides long and are found in nearly all eukaryotic cells. MIRs regulate gene activity by imperfect base pairing to the 3' UTR of their target mRNAs, leading to mRNA degradation or inhibition of translation. One single MIR may regulate expression of as many as 200 different gene targets, which means that expression of about one-third of human mRNAs might be controlled by MIRs. Altered expression of MIRs can therefore contribute to malignant transformation by increasing or decreasing expression of oncogenes and tumour suppressor genes, respectively (reviewed by Calin and Croce, Nat Rev Cancer 2006, 6, 857-866). The fact that expression profiling of a limited amount of MIRs provided a very accurate classifier of tumour subtypes, yielding even better results than a classifier based on genome-wide mRNA expression profiling, underlines the importance of miRNAs in carcinogenesis in general (Lu et al., Nature 2005;435:834-8). Interestingly, miRNA expression levels in tumours are globally lower compared to normal tissue. Like for protein encoding tumor suppressor genes, also silencing of miRNAs may results from epigenetic alterations, such as DNA methylation of regulatory sequences.

Most microRNA genes are found in intergenic regions or in anti-sense orientation to genes and contain their own miRNA gene promoter and regulatory units.

The *MAL* (T-lymphocyte maturation associated protein) gene (Genbank Accession NM_002371.2) has originally been identified as a differentially expressed gene during T-cell development (Alonso and Weisman 1987, Proc.Natl.Acad.Sci. USA, 84, 1997-2001). MAL encodes a 17kDa integral membrane protein and is a component of glycolipid enriched membrane microdomains or rafts (Kim et al. 1995, J.Neurosci.Res.,42, 413-422). MAL has an essential role as a component of the protein machinery for apical transport of membrane and secretory proteins in polarized epithelial cells (Cheong et al., 1999, Proc.Natl.Acad.Sci. USA, 84, 6241-6248).

Reduced MAL expression has been detected in a various number of human cancers, including esophageal, gastric and colorectal cancer (Mimori et al., 2003, Oncogene, 22, 3463-3471; Mimori 2007 et al., Ann. Surg. Oncol.,14, 1670-1677)

In colorectal cancer MAL downregulation has been associated with promoter hypermethylation (Mori et al, 2006, Gastroenterology, 131,797-808; Lind et al., 2007 Gastroenterology, 132, 1631-1632)

A functional role of MAL acting as a tumor suppressor gene was demonstrated by re-expression of the MAL gene in esophageal cancer cells, resulting in a suppression of motility, invasion and tumorigenicity, while enhancing apoptosis (Mimori et al., 2003, Oncogene, 22, 3463-3471)

It was established in WO 2009/128714 that alterations in *MAL* promoter methylation are frequently observed in cervical carcinomas of both squamous cell carcinoma, adeno-squamous carcinoma, adenocarcinoma and neuroendocrine carcinoma histotypes, and their high-grade precursor lesions. *In vitro* studies revealed a functional involvement of *MAL* inactivation in cervical cancer development, as MAL overexpression in cells of the HPV 16 containing SiHa cervical cancer cell line reduced proliferation and suppressed anchorage independent growth. Most interestingly, the present inventors have therein shown that hypermethylation of the MAL promoter could be detected in cervical-vaginal specimens collected by self-sampling and was found to be associated with the presence of an underlying high-grade CIN lesion or invasive cervical cancer.
Interestingly, as shown in the experimental part of the present invention, by combining methylation analysis of MAL with methylation analysis of a promoter region of the microRNA miR124-2 a high sensitivity for ≥CIN 2 is reached.
These results indicate that the detection of MAL promoter methylation in combination with hsa-miR-124 promoter methylation in self-collected cervical-vaginal specimens can predict high-grade CIN disease or cervical cancer.

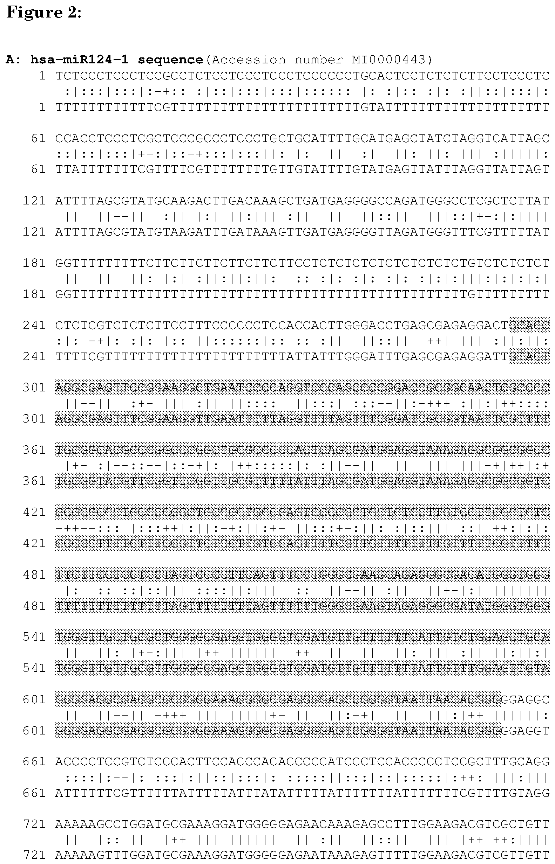

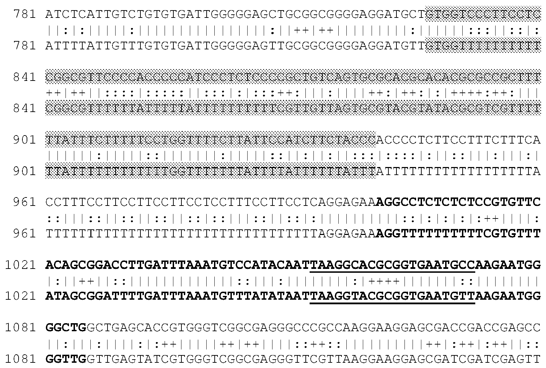

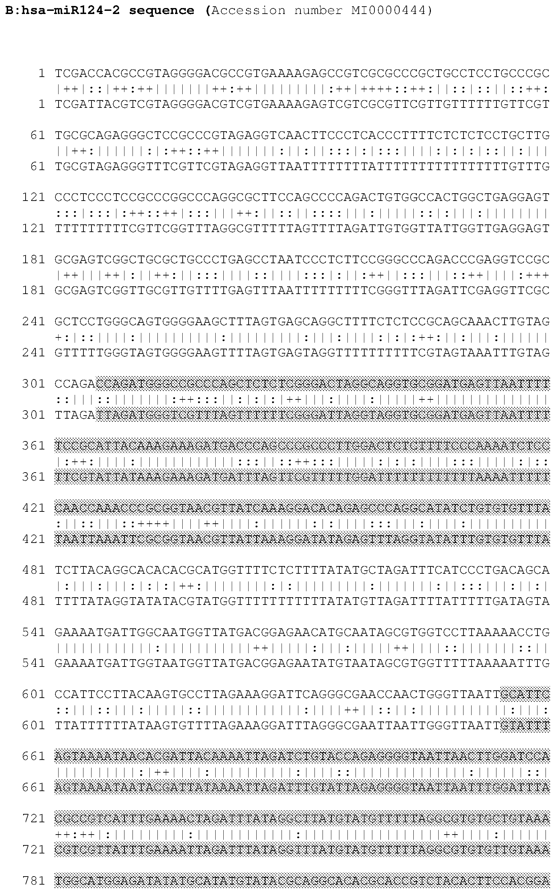

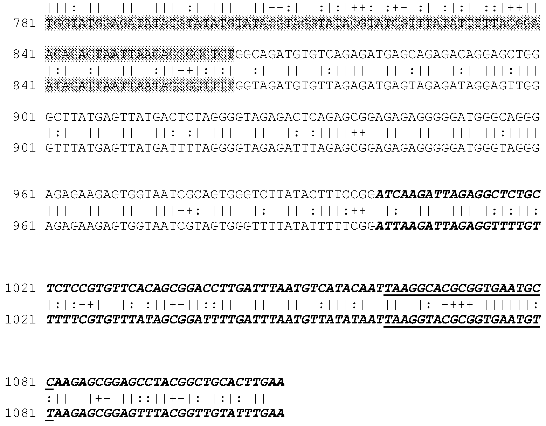

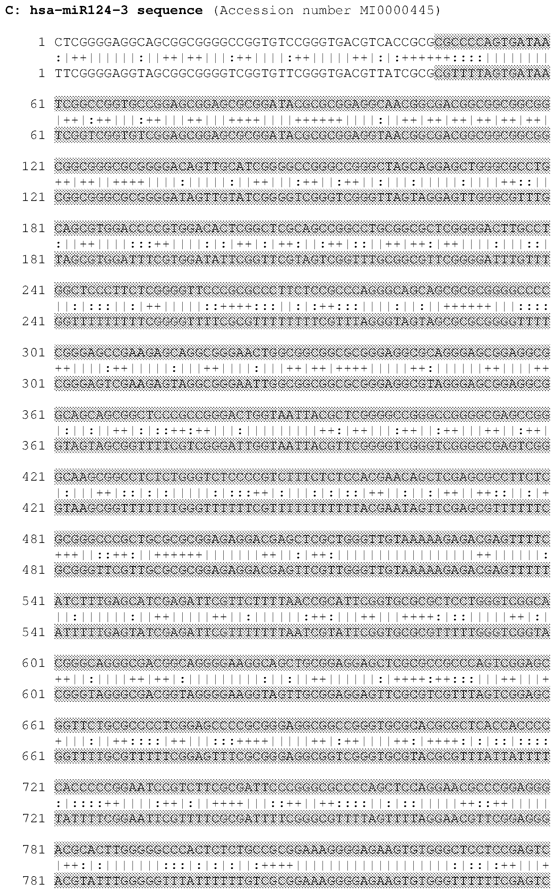

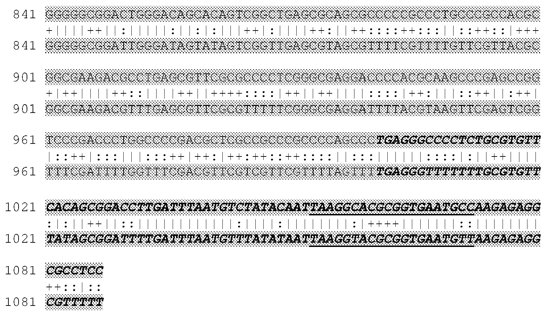

The mature hsa-m*iR124* sequence (Accession number MIMAT0000422) is encoded by 3 individual premature microRNA sequences located at three different genomic regions, i.e. hsa-miR124-1 (Accession number MI0000443) at chromosome 8p23.1, hsa-miR124-2 (Accession number MI0000444) at chromosome 8q12.3 and hsa-miR124-3 (Accession number MI0000445) at chromosome 20q12.33 (Accession numbers as indicated on the miRNA database, miRBase (www.mirbase.org), Faculty of Life Sciences, University of Manchester) . Although the mature sequence is exactly the same, the premature and regulatory sequences are different (see Fig. 2). If, in this application, the term hsa-miR124 is used, any form of hsa-miR124 is included.
Epigenetic down-regulation of hsa-miR124 expression has already been described in several human cancers, including gastric cancer, colon, breast and lung carcinomas and acute lymphoblastic leukaemia (Lujambio et al., Cancer Res 2007, 67, 1424-1429; Andro IntJ Cancer 2009; 124: 2367-2374, Agirre et al. Cancer Res 2009;69:4443-4453). Previous studies by Lujambio *et al.* and Agirre *et al.* have already pointed out a putative tumour suppressor function for hsa-miR124. In both studies, epigenetic silencing of hsa-miR124 expression was demonstrated to mediate up-regulation of cyclin D kinase 6 (CDK6), one of the predicted targets of hsa-miR124. CDK6 is known to be involved in cell cycle progression and differentiation and can inactivate the retinoblastoma protein (pRb), a well-known tumour-suppressor, in order to promote cell growth.

Accordingly, the present invention provides methods of detecting the occurrence of HPV-induced high-grade precancerous lesions and HPV-induced invasive cancers comprising assaying a cervico-vaginal lavage for the presence of alteration in methylation of T-lymphocyte maturation associated protein (MAL) and hsa-miR124, wherein said alteration indicates the presence of HPV-induced precursor lesions with invasive potential and HPV-induced invasive cancers. Said presence of high-grade precursor lesions and/or invasive cancers shows by an increased methylation (also called hypermethylation) of the MAL gene and especially the promoter region thereof and the hsa-miR124 promoter region.

The self-sample of the subject will contain mucosal cells, such as cervical cells, wherein a precursor lesion or cancer associated with HPV is to be detected.

A method of the present invention is particularly suited for the detection of high-grade precancerous lesions and invasive cancers that are induced by high-risk HPVs. Preferably, the assay method of the invention is performed on samples which have already been classified as hrHPV positive.

Figure 1 shows the CpG-rich promoter region and CpG-rich first intronic sequence of the MAL gene as well as the coding sequence and transcribed 3' non-coding sequence. Methylation of the CpG-rich sequences particularly in the promoter region will result in a sharply decreased transcription or even complete blockage of transcription. A similar phenomenon plays a role in hsa-miR124, of which the sequence is depicted in Fig. 2. Here also CpG rich areas are indicated and methylation of these will result in a sharply decreased transcription or even complete blockage of transcription of the hsa-miR124 molecule. The assay is preferably conducted on the nucleic acid content of the sample, where the reagent is typically a nucleic acid (DNA or RNA) probe or (PCR) primer. By using such probes or primers, gene expression products, such as mRNA may for example be detected. The reagent may also be a restriction endonuclease, preferably a methylation sensitive restriction endonuclease for the detection of the presence of methyl groups on the sample nucleic acid, said sample nucleic acid then preferably being DNA.

The sample nucleic acid may be detected directly *in situ* or it may be isolated from other cell components by common methods known to those of skill in the art before contacting with the reagent (see for example, "Current Protocols in Molecular Biology", Ausubel et al. 1995. 4th edition, John Wiley and Sons; "A Laboratoty Guide to RNA: Isolation, analysis, and synthesis", Krieg (ed.), 1996, Wiley-Liss; "Molecular Cloning: A laboratory manual", J. Sambrook, E.F. Fritsch. 1989. 3 Vols, 2nd edition, Cold Spring Harbor Laboratory Press).

It appears that the methylation status of MAL and hsa-miR124 is predictive for the presence of high-grade precursor cervical lesion or cervical invasive cancers. Hypermethylation occurs in particular in the cytosine rich areas termed "CpG islands", which are primarily situated in the 5' regulatory regions of genes and which are normally unmethylated. The term "hypermethylation" includes any methylation of cytosine at a position that is normally unmethylated in the *MAL* gene sequence (e. g. the *MAL* promoter, first exon and first intronic sequence, see Figure 1) or the hsa-miR124 sequence (Fig. 2). Hypermethylation can for instance be detected by restriction endonuclease treatment of the *MAL* and hsa-miR124 polynucleotide (gene) and Southern blot analysis. Therefore, in one embodiment of the method of the invention, restriction endonuclease analysis is preferred to detect hypermethylation of the *MAL* gene or hsa-miR124 sequence. Any restriction endonuclease that includes CG as part of its recognition site and that is inhibited when the C is methylated, can be utilized. Methylation sensitive restriction endonucleases such as BssHII, MspI, NotI or HpaII, used alone or in combination, are examples of such endonucleases. Other methylation sensitive restriction endonucleases will be known to those of skill in the art.

Other methods for the detection of *MAL* gene or hsa-miR124 sequence hypermethylation involve bisulfite modification of DNA, in which the unmethylated cytosines are converted to an uracil whereas the methylated cytosines are protected from chemical modification. Subsequent PCR amplification and sequencing, including pyrosequencing will reveal whether cytosines in CpG islands are maintained in case of methylation or replaced by a uracil in case of an unmethylated status. Another method involves the treatment of a PCR amplified product generated from bisulfite modified DNA with a restriction endonuclease that includes CG as part of its recognition site.

An alternative means to test for methylated sequences is a methylation specific PCR, which is also based on bisulfite modification of DNA, followed by specific PCR reactions that target CpG rich sequences.

For purposes of the invention, a-nucleic acid probe specific for MAL or hsa-miR124 may be used to detect the presence of *MAL* or hsa-miR124 polynucleotide (using nucleic acid probe) in biological fluids or tissues. Oligonucleotide primers based on any coding sequence region and regulatory sequence region in the *MAL* or hsa-miR124 sequence are useful for amplifying DNA, for example by PCR.

When using PCR primers, nucleic acid probes or restriction endonucleases, the 5' regulatory region, first intronic sequence and coding sequence of the *MAL* sequence (as specified in Figure 1) or the hsa-miR124 sequence (as specified in Fig. 2) is analysed.

In one embodiment of a method of the invention an increased methylation of the *MAL* gene (including the promoter region) and the hsa-miR124 sequence in the test cell is detected as compared to the comparable normal cell.

The present invention also provides the use of a kit of parts in a method of detecting HPV-induced precursor lesions with invasive potential and HPV-induced invasive cancers according to the invention. Such a kit may suitably comprise a sampling device consisting of an irrigation syringe, a disposable female urine catheter and a container with irrigation fluid will be included to collect cervico-vaginal cells by a lavage. A kit according to the present invention may comprise primers and probes for the detection of MAL and hsa-miR124-2 methylation. Primers and probes as used for the present invention are given in Table 1 and Table 2.
Table 1: MAL Primer-probe combinations used for quantitative methylation specific PCR analysis on self-samples:

| | |
|---|---|
| **MAL forward primer** | F: GCGTAGTATTAAGTAGAGAGGTTCG |
| **MAL reverse primer** | R: AATAAAAAATAAAACCGACCGC |
| **MAL probe** | P: ACTAAACCGACGCTAATTCGACGCT |

A kit of parts as disclosed herein comprises means for the detection of MAL and hsa-miR124 methylation, such as methylation-sensitive restriction enzymes, or probes, primers specific for MAL or specific for the MAL nucleotide sequence of Figure 1 or probes, primers specific for hsa-miR124.

In yet another alternative embodiment of a kit as used herein the means for the detection of MAL and hsa-miR124 methylation may be combined with means for the detection of HPV infection, preferably for the detection of HPV infection of the high-risk type. Such means may comprise HPV-specific primers or probes, protein markers for HPV infection or even surrogate markers for HPV infection as are known in the art.

### EXAMPLES

### Example 1. Hsa-miR124 methylation in HPV-immortalized keratinocyte cell lines and cervical cancer cell lines

Since the mature hsa-miR124 sequence is encoded at three different genomic locations (hsa-miR124-1 [8p23.1], hsa-miR124-2 [8q12.3] and hsa-miR124-3 [20q13.33]), the hsa-miR124 methylation status was determined at all 3 loci by quantitative methylation specific PCR (MSP). The following amplicons were detected: hsa-miR124-1: nt 811 to 904; hsa-miR124-2: nt 701 to 838; hsa-miR124-3: nt 897 to 991, each referring to the sequences shown in Figure 2. The housekeeping gene B-actin was used as an internal reference (Harden et al., J Urology 2003;169:1138-1142). Using these assays we found no hsa-miR124 methylation in primary keratinocytes isolated from three different donors (EK), whereas the corresponding CpG islands of all three genomic loci were methylated in three hrHPV-containing cervical carcinoma cell lines (SiHa, HeLa and CaSki). In early and late passages of four hrHPV-immortalized keratinocyte cell lines (FK16A, FK16B, FK18A and FK18B), representing consecutive stages of HPV-induced malignant transformation (Steenbergen et al., Oncogene 1996;13:1249-1257), both hsa-miR124-1 and hsa-miR124-2 were frequently methylated, although methylation levels were relatively low in early passages (passage 23-43) as compared to high methylation levels in late passages (passage 70-96). The third gene, hsa-miR124-3, was not methylated in any of the HPV-immortalized cell lines. Whereas hsa-miR124 RNA expression was undetectable in SiHa cervical cancer cells, we did detect hsa-miR124 re-expression in SiHa cells treated with the DNA-demethylating agent 5-aza-2'-deoxycytidine (DAC, 5 µM). This indicates that hsa-miR124 gene silencing is (in)directly mediated by epigenetic modifications, predominantly DNA methylation, in SiHa cells.

### Example 2: hsa-miR124 overexpression in SiHa cells has antiproliferative and anti-migratory effects

To determine the possible functional relevance of decreased hsa-miR124 expression in cervical carcinogenesis, two cervical cancer cell lines SiHa and CaSki were transduced with retroviral vectors that contained gene constructs leading to stable expression of hsa-miR124. Expression analysis using RT-PCR confirmed ectopic expression of hsa-miR124 in hsa-miR124 transductants and absence of expression in cells transduced with an empty retroviral vector. Ectopic expression of hsa-miR124 in both SiHa and CaSki transductants resulted in a significant decrease of cellular proliferation (p<0.05) in comparison with hsa-miR-ctrl transductants following 6 days of culturing. Moreover, using a wound-healing assay, SiHa hsa-miR124 transductants were found to have a decreased migratory capacity as compared with SiHa hsa-miR-ctrl cells.

### Example 3: hsa-miR124 promoter methylation in cervical tissue specimens

To determine whether and when DNA hypermethylation of all three hsa-miR124 regulatory regions occurs during the process of cervical carcinogenesis *in vivo*, we performed quantitative MSP analysis, using primers and probes as defined in Table 2 on cervical tissue specimens obtained from normal cervix, low-grade CIN lesions (CIN1), high-grade CIN lesions (CIN3) and cervical carcinomas (both SCCs and AdCas). In normal cervix, 6% (1/18) samples scored positive for methylation at hsa-miR124-3, while in none of the normal samples methylation at the other two gene regulatory regions (hsa-miR124-1 and hsa-miR124-2) was detected. In CIN1 lesions, 28% (10/36) showed methylation at hsa-miR124-1, 6%(2/36) at hsa-miR124-2 and 11% (4/36) at hsa-miR124-3. Methylation positivity in CIN3 lesions varied from 46% (19/41) for hsa-miR124-1, 20% (8/41) for hsa-miR124-2 to 10% (4/41) for hsa-miR124-3. Frequencies of hsa-miR124-1, hsa-miR124-2 and hsa-miR124-3 as detected in SCCs were 86% (25/29), 83% (24/29) and 72% (21/29), respectively. In case of AdCa, methylation of hsa-miR124-1, hsa-miR124-2 and hsa-miR124-3 was detected in 93% (14/15), 80% (12/15) and 73% (11/15) respectively, of these tumours. When combining the three MSP assays it was found that the combination of hsa-miR124-1 and hsa-miR124-2 has the highest combined sensitivity and specificity for ≥CIN3, as all normal cervical epithelial samples test negative, and 59% of CIN3 lesions, 93% of SCCs and 93% of AdCas are test positive. It should be noted that above frequencies account for tissue specimens only with thresholds set at a specificity of 100%. Lowering the threshold increases the sensitivity for CIN3, though lowers the specificity.

**Table 2: Primers and probes for quantitative methylation specific PCR analysis of MIR124-1, MIR124-2 and MIR124-3.**

| | Forward primer | Probe | Reverse primer | Location amplicon | Amplico n length |
|---|---|---|---|---|---|
| MIR124-1 | 5-CGGCGGGGAGGATGTT -3' | 5'- CGGCGTTTTTTATTTTT- 3' | 5'- ATAAAAAACGACGCGTATACGTACG-3' | 811-904 | 94 bp |
| MIR124-2 | '5-GGGTAATTAATTTGGATTTACGTCGTTAT-3' | 5'- TTTACAACACACGCCTAAA - 3' | 6'-CGTAAAAATATAAACGATACGTATACCTACGT-3' | 701-838 | 138 bp |
| MIR124-3 | 5'-ACGCGGCGAAGACGTTT-3' | 5'- AAAATCCTCGCCCGAAAAACGCGA- 3' | 5'- CGAACGACGAACGTCGAAA-3' | 897-991 | 95 bp |

### Example 4 : hsa-miR124 methylation in cervical scrapings

To assess the putative marker value of hsa-miR124 methylation in clinical practice, we assessed hsa-miR124 in cervical scrapes.

Using a nested case-control design of women participating in a population-based screening trial we studied cervical scrapes of hrHPV positive women in which ≥CIN 2 (including 1 carcinoma) was diagnosed within 18-months of follow-up (i.e., cases) versus hrHPV positive women in whom at maximum CIN 1 was diagnosed within an 18-month follow-up period (i.e., controls). Hsa-miR124-1 methylation was detected in 5% of controls and 48% of cases. Hsa-miR124-2 methylation was absent in controls and detected in 48% of cases and hsa-miR124-3 methylation was detected in 5% of controls and 24% of cases. Combined analysis of hsa-miR124-1 and hsa-miR124-2 detected 5% controls and 71% of cases. Further adjustments of threshold settings increased the sensitivity rates for CIN3 to >80%, with specificity rates still being acceptable (>50%).

### Example 5: hsa-miR124 methylation in self sampled specimens

We subsequently analysed self-sampled cervico-vaginal specimens collected using either a VibaBrush (Rovers Medical Devices, Oss, the Netherlands) or a Pantarhei sampler (Pantarhei Devices, Zeist, The Netherlands) during the course of a prospective study in which a total of 45,000 self-sampling packages were be sent to women who, even after a second reminder, did not respond to the invitation for regular cervical screening (See www.trialregister.nl, Trial no.NTR962 (PROHTECT trial)). About one third of these women returned self-sampled specimens to the lab. These samples are suitable for HPV PCR analysis (i.e. beta-globin PCR positive) and testing by hrHPV GP5+/6+-PCR yields at least as much ≥CIN 2 lesions in this population as found by regular screening in a matched population of responder women (Gök et al.2010 Brit.Medical J.;340:c1040).

55% of self-samples of women who later were diagnosed with ≥CIN3 tested positive for either of the MIR124 markers, compared with only 25% of women without evidence of clinically meaningful disease in follow-up. These data show that MIR124 methylation analysis on self-sampled materials is well feasible and will improve the detection of underlying high-grade cervical disease.

### Example 6: Comparison of the performance of CADM1 and MAL methylation analysis on self-collected cervico-vaginal lavage specimens to their performance on physician-collected cervical scrapes

Combined testing for promoter methylation of the gene encoding T-lymphocyte maturation associated protein, also known as T-cell differentiation protein (further referred to as *MAL*; Genbank Accession NM_002371) and the gene encoding cell adhesion molecule 1 (CADM1) appeared an attractive alternative molecular triage tool when applied to physician-collected cervical scrapes that performed equally well as cytology (Hesselink et al. 2011, Clin Cancer Res; 17(8):2459-65). As such, we next evaluated MAL and CADM1 promoter methylation on hrHPV DNA-positive self-collected cervico-vaginal lavage specimens obtained from non-attendees who participated in the PROHTECT cohort study [Gök et al. 2010Brit.Medical J.;340:c1040] and compared their performance to the results obtained in physician-collected cervical scrapes (n=236) as described by Hesselink et al.(Hesselink et al. 2011, Clin Cancer Res; 17(8):2459-65).

Of the 757 women who tested positive for hrHPV on their self-collected cervico-vaginal lavage specimen in the PROHTECT 1 trial, 355 women ultimately had a valid study endpoint. These comprised 74 women who had been diagnosed with CIN3+, including four squamous cell carcinomas and one adenocarcinoma, 20 with CIN2, and 261 with no evidence of CIN2+ (i.e., at maximum CIN1). The latter group is hereafter referred to as ≤CIN1. All samples were tested for both MAL (region M1) and CADM1 (region M18) methylation using quantitative methylation specific PCR according to the same criteria as previously described for the physician-collected cervical scrapes (Hesselink et al. 2011, Clin Cancer Res; 17(8):2459-65)

MAL methylation analysis was found to perform equally well on self-collected cervico-vaginal lavage specimens compared to physician-collected cervical scrapes, both with respect to the detection of CIN3+ as CIN2+ lesions (Figure **3A**). In contrast, the signals obtained upon CADM1 methylation analysis of self-collected cervico-vaginal lavage specimens were markedly reduced compared to those obtained in physician-collected cervical scrapes and as such particularly the detection of CIN3+ in self-collected cervico-vaginal lavage specimens was unsatisfactory (**Figure 3B**).

This discrepancy most likely reflects the fact that self-collected specimens may have a different cellular composition (i.e., relative deficiency of cervical cells compared to vaginal cells) than cervical smears taken by a physician. Accordingly, the most optimal triage markers for hrHPV-positive physician-taken cervical scrapes may not necessarily be directly translated to self-samples. For an optimal clinical performance a different panel of methylation markers is likely to be required.

### Example 7: Analysis of hsa-miR124-2 methylation in combination with MAL methylation in self-collected cervico-vaginal lavage specimens

As described in Example 1, there is a need for an alternative methylation marker to enhance the clinical performance of methylation analysis in self-collected specimens. Our previous studies using MAL and CADM1 have shown that markers representing distinct functional events in the multistep process of cervical carcinogenesis can be complementary to each other in terms of CIN3+ detection (Overmeer et al., Int J Cancer 2010 Epub Dec 28,; Hesselink et al. 2011, Clin Cancer Res; 17(8):2459-65). Hence, we hypothesized that for analysis of self-collected specimens an alternative marker representing the same stage of transformation as CADM1 is to be preferred. Whereas MAL methylation occurs rather early during transformation, i.e. high methylation levels in early passages of HPV-immortalized cells, and CADM1 methylation is linked to later stages of transformation, such as anchorage independent growth and tumorigenicity, we sought for methylation events functionally associated with later stages of transformation. One such event is methylation-mediated silencing of human microRNA 124. High methylation levels of the genes encoding these microRNAs became apparent in late passages of HPV-transfected keratinocytes upon the acquisition of anchorage independent growth characteristics. Moreover, overexpression of hsa-miR124 was found to suppress the oncogenic growth properties of cervical cancer cells, supporting a functional role in cervical carcinogenesis, see Example 2.

The methylation marker hsa-miR124 was therefore evaluated for its potential to complement the MAL marker for detection of CIN3+in the same series of hrHPV-positive self-collected specimens as tested for CADM1 and MAL (see Example 1). Of interest, analysis of hsa-miR124-2 revealed a much better discrimination of CIN3+ lesions compared to CADM1 (Figure 4). Most importantly, the hsa-miR142-2/MAL combination was found to have an excellent clinical performance on self-collected specimens (i.e. CIN3+ sensitivity of 86.5% at 52.5% specificity), equalling that of CADM1/MAL in physician-collected samples (i.e.CIN3+ sensitivity of 83.3% at 50% specificity).

All primer and probe combinations used for qMSP analysis on self-collected cervico-vaginal specimens are listed in Table 1 and Table 2.

## Claims

1. A method of detecting the occurrence of HPV-induced high-grade precancerous cervical lesions and/or HPV-induced invasive cervical cancers comprising assaying a cervico-vaginal lavage sample for the presence of an alteration in methylation of T-lymphocyte maturation associated protein (MAL) and hsa-miR124, wherein said alteration indicates the presence of HPV-induced precursor lesions with invasive potential and/or HPV-induced invasive cancers.

2. Method according to claim 1, wherein said HPV-induced invasive cancer is a high-risk HPV-induced invasive cancer.

3. Method to any of the preceding claims wherein said alteration is an increase in methylation in comparison to a normal, control sample.

4. Method according to any one of the preceding claims, wherein the nucleic acid is DNA.

5. Method according to claim 4, wherein the assay is performed with a restriction endonuclease, preferably a methylation sensitive restriction endonuclease.

6. Method according to claim 4, wherein the reagent is a nucleic acid probe or primer that binds to the nucleic acid.

7. Method according to claim 6, wherein said nucleic acid probe or primer has a detectable label.

8. Method according to claim 6 or 7, wherein the nucleic acid probe has a nucleotide sequence selected from the group consisting of the sequences depicted in Table 1.

9. Use of a molecular diagnostic marker set for the detection of HPV-induced high-grade precancerous lesion or HPV-induced invasive carcinoma in a cervico-vaginal lavage sample, wherein said marker set indicates alteration in methylation of the MAL gene or promoter and alteration in methylation of the hsa-miR124 sequence.

10. Use of a kit of parts in a method of detecting HPV-induced high-grade precancerous lesion or HPV-induced invasive carcinoma in test cells from a cervico-vaginal lavage sample of a subject, said kit comprising a methylation sensitive restriction endonuclease and
- means for the detection of methylation of the MAL gene or promoter wherein said means comprise probes, primers specific for MAL or specific for the MAL nucleotide sequence of Figure 1; and
- means for the detection of hsa-miR124 methylation, wherein said means comprise probes, primers specific for hsa-miR124.

11. Use according to claim 10, wherein said kit of parts further comprises means for the detection of HPV infection, wherein said means comprise probes and primers specific for HPV.

12. Use of a kit of parts as defined in claims 10 or 11, further comprising means for taking a cervico-vaginal lavage.

## Patentansprüche

1. Verfahren zum Nachweis des Auftretens von HPV-induzierten hochgradig präkanzerösen Zervixläsionen und/oder HPV-induzierten, invasiven Zervixkarzinomen, umfassend Untersuchen einer zervikovaginalen Spülungsprobe auf das Vorhandensein einer Veränderung in der Methylierung von mit der Reifung von T-Lymphozyten verbundenem Protein (engl. methylation of T-lymphocyte maturation associated protein, MAL) und hsa-miR124, wobei die Veränderung auf das Vorhandensein von HPV-induzierten Vorläuferläsionen mit invasivem Potenzial und/oder HPV-induzierten Karzinomen hinweist.

2. Verfahren nach Anspruch 1, wobei das HPV-induzierte invasive Karzinom ein HPV-induziertes invasives Karzinom mit hohem Risiko ist.

3. Verfahren nach einem der vorhergehenden Ansprüche, wobei die Veränderung eine Zunahme der Methylierung im Vergleich zu einer normalen Kontrollprobe ist.

4. Verfahren nach einem der vorhergehenden Ansprüche, wobei die Nukleinsäure DNA ist.

5. Verfahren nach Anspruch 4, wobei der Test mit einer Restriktionsendonuklease, bevorzugt einer methylierungssensitiven Restriktionsendonuklease durchgeführt wird.

6. Verfahren nach Anspruch 4, wobei das Reagens eine Nukleinsäuresonde oder Primer ist, die/ der an die Nukleinsäure bindet.

7. Verfahren nach Anspruch 6, wobei die Nukleinsäuresonde oder Primer eine nachweisbare Markierung hat.

8. Verfahren nach Anspruch 6 oder 7, wobei die Nukleinsäuresonde eine Nukleotidsequenz hat, ausgewählt aus der Gruppe bestehend aus den Sequenzen dargestellt in Tabelle 1.

9. Verwendung eines molekularen diagnostischen Markersets zum Nachweis von HPV-induzierter hochgradig präkanzeröser Läsion oder von HPV-induziertem invasivem Karzinom in einer zervikovaginalen Spülungsprobe, wobei das Markerset auf Veränderung in der Methylierung des MAL-Gens oder -Promotors und eine Veränderung in der Methylierung der hsa-miR124-Sequenz hinweist.

10. Verwendung eines Kits von Teilen in einem Verfahren zum Nachweis von HPV-induzierter hochgradig präkanzeröser Läsion oder HPV-induziertem invasivem Karzinom in Testzellen von einer zervikovaginalen Spülungsprobe eines Subjekts, das Kit umfassend eine methylierungssensitive Restriktionsendonuklease und
- Mittel für den Nachweis von Methylierung des MAL-Gens oder - Promotors, wobei die Mittel Sonden, Primer spezifisch für MAL oder spezifisch für die MAL-Nukleotidsequenz von Abbildung 1, umfassen; und
- Mittel für den Nachweis von hsa-miR124-Methylierung, wobei die Mittel Sonden, Primer spezifisch für hsa-miR124 umfassen.

11. Verwendung nach Anspruch 10, wobei das Kit von Teilen ferner Mittel für den Nachweis von HPV-Infektion umfasst, wobei die Mittel Sonden und Primer spezifisch für HPV umfassen.

12. Verwendung eines Kits von Teilen wie in den Ansprüchen 10 oder 11 definiert, ferner umfassend Mittel zur Entnahme einer zervikovaginalen Spülung.

## Revendications

1. Méthode de détection de l'apparition de lésions cervicales précancéreuses induites par VPH et/ou de cancers cervicaux invasifs induits par VPH, comprenant l'étape consistant à doser un échantillon de lavage cervico-vaginal pour la présence d'une altération de méthylation d'une protéine associée à la maturation de lymphocytes T (MAL) et de hsa-miR124, dans laquelle ladite altération indique la présence de lésions précurseurs induites par VPH avec un potentiel invasif et/ou des cancers invasifs induits par VPH.

2. Méthode selon la revendication 1, dans laquelle ledit cancer invasif induit par VPH est un cancer invasif induit par VPH à haut risque.

3. Méthode selon l'une quelconque des revendications précédentes, dans laquelle ladite altération est une augmentation de méthylation par rapport à un échantillon témoin normal.

4. Méthode selon l'une quelconque des revendications précédentes, dans laquelle l'acide nucléique est l'ADN.

5. Méthode selon la revendication 4, dans laquelle le dosage est effectué avec une endonucléase de restriction, de préférence une endonucléase de restriction sensible à la méthylation.

6. Méthode selon la revendication 4, dans laquelle le réactif est une sonde ou amorce d'acide nucléique qui se lie à l'acide nucléique.

7. Méthode selon la revendication 6, dans laquelle ladite sonde ou amorce d'acide nucléique possède une étiquette détectable.

8. Méthode selon la revendication 6 ou 7, dans laquelle la sonde d'acide nucléique a une séquence nucléotidique sélectionnée dans le groupe constitué des séquences indiquées dans le Tableau 1.

9. Utilisation d'un ensemble de marqueurs de diagnostic moléculaire pour la détection d'une lésion précancéreuse induite par VPH ou d'un carcinome invasif induit par VPH dans un échantillon de lavage cervico-vaginal, dans laquelle ledit ensemble de marqueurs indique une altération de méthylation du gène ou promoteur MAL et une altération de méthylation de la séquence hsa-miR124.

10. Utilisation d'un kit de pièces dans une méthode de détection d'une lésion précancéreuse induite par VPH ou d'un carcinome invasif induit par VPH dans des cellules de test à partir d'un échantillon de lavage cervico-vaginal d'un sujet, ledit kit comprenant une endonucléase de restriction sensible à la méthylation et
- des moyens pour la détection de la méthylation du gène ou promoteur MAL, dans laquelle lesdits moyen comprennent des sondes, des amorces spécifiques pour MAL ou spécifiques pour la séquence nucléotidique MAL de la figure 1 ; et
- des moyens pour la détection de la méthylation de hsa-miR124, dans laquelle lesdits moyens comprennent des sondes, des amorces spécifiques pour hsa-miR124.

11. Utilisation selon la revendication 10, dans laquelle ledit kit de pièces comprend en outre des moyens pour la détection d'une infection par VPH, dans laquelle lesdits moyens comprennent des sondes et des amorces spécifiques pour VPH.

12. Utilisation d'un kit de pièces selon la revendication 10 ou 11, comprenant en outre des moyens pour la prise d'un lavage cervico-vaginal.
